# EUROPÄISCHE PATENTSCHRIFT

(11) **EP 2 479 164 B1**
(45) Veröffentlichungstag und Bekanntmachung des Hinweises auf die Patenterteilung: **13.11.2013**
(21) Anmeldenummer: 12002597.8
(22) Anmeldetag: 02.05.2007
(51) Int. Cl.: C07C 51/235, C07C 53/126

(54) **Katalytisches Verfahren zur Herstellung von aliphatischen geradkettigen und beta-alkylverzweigten Carbonsäuren**
Catalytic method for producing aliphatic straight chain and beta-alkyl branched carboxylic acids
Procédé catalytique destiné à la fabrication d'acides carboniques aliphatiques, à chaînes linéares et ramifiés en bêta alkyles

(30) Priorität: 12.05.2006 DE 102006022168
(43) Veröffentlichungstag der Anmeldung: 25.07.2012
(62) Teilanmeldung aus: 07008849.7
(73) Patentinhaber: OXEA GmbH, 46147 Oberhausen (DE)
(72) Erfinder: Springer, Helmut, 46539 Dinslaken (DE)

(56) Entgegenhaltungen:
- DE-C1- 10 010 771
- GB-A- 856 962

## Beschreibung

Die vorliegende Erfindung betrifft ein neues, katalytisches Verfahren zur Herstellung von aliphatischen geradkettigen und β-alkylverzweigten Carbonsäuren aus Aldehyden durch Oxidation mit Sauerstoff oder Sauerstoff enthaltenden Gasen.

Aldehyde werden in großem Umfang als Ausgangsstoffe für die Herstellung von Carbonsäuren eingesetzt. Die Vorzugsstellung für diese Verwendung verdanken Aldehyde ihrer hohen Verfügbarkeit nach einer Reihe, auch industriell genutzter Prozesse. Überdies läßt sich die Carbonylgruppe der Aldehyde leicht in die Carboxylgruppe umwandeln. Im Rahmen technisch ausgeübter Verfahren erfolgt die Umsetzung von Aldehyden zu Carbonsäuren vorwiegend in Anwesenheit von Katalysatoren. Als Katalysatoren kommen vorwiegend Salze von Übergangsmetallen in Betracht, insbesondere Salze des Kobalts und des Mangans sowie des Chroms, Eisens, Kupfers, Nickels, Silbers und Vanadiums. Dennoch ist die Carbonsäurebildung aus Aldehyden, selbst bei Einhaltung optimaler Temperaturbedingungen, häufig mit Neben- und Abbaureaktionen verbunden.

J. Prakt. Chem. Bd. 14 (1961), 71-83 beschreibt die Oxidation von Isononanal in Gegenwart von Kobaltacetat oder Mangannaphthenat. In Gegenwart des Mangan enthaltenden Katalysators beträgt bei einer Reaktionstemperatur von 60°C die Ausbeute an Isononansäure nur ca. 70%.

Nach dem in der DE-OS 30 29 700 beschriebenen Verfahren werden zur Herstellung von aliphatischen Monocarbonsäuren mit 6 bis 9 Kohlenstoffatomen die entsprechenden Aldehyde mit Sauerstoff in reiner Form oder mit Luft oxidiert. Als Katalysator wirkt eine Kombination von Mangan- und Kupferverbindungen, die in der Säure löslich sind. Die Metalle liegen in einer Menge von je etwa 10 bis etwa 2000 ppm, vorzugsweise 200 bis 600 ppm Mangan und Kupfer, bezogen auf das Gewicht des flüssigen Reaktionsgemischs, vor. Das Molverhältnis von Mangan zu Kupfer beträgt 5:1 bis 0,5:1. Die Umsetzung der Ausgangsstoffe erfolgt in flüssiger Phase bei Temperaturen von etwa 50 bis 80°C und Drücken im Bereich von etwa 1,4 bis 10,3 bar. Als Hauptschwierigkeit dieses Verfahrens wird in der Prozessbeschreibung die Anwesenheit von Kupfer- und auch Manganverbindungen im Reaktionsprodukt, d.h. in der Carbonsäure, geschildert. Zur Entfernung der Metalle sind aufwendige Reinigungsmaßnahmen erforderlich, beispielsweise ihre Ausfällung mit wässriger Oxalsäure.

Das in der US-Patentschrift 4 487 720 offenbarte Verfahren zur Herstellung von C₅- bis C₉-Monocarbonsäuren durch Oxidation von Aldehyden der gleichen Kohlenstoffatom-Zahl mit reinem Sauerstoff oder mit Luft arbeitet ebenfalls mit Kupfer- und Manganverbindungen als Katalysatoren. Die Gesamtmenge der Metalle erstreckt sich über einen Bereich von 10 bis 200 ppm, bezogen auf das Gesamtgewicht der aus Aldehyd, Säure und Katalysator bestehenden Lösung. Mangan und Kupfer werden in einem Molverhältnis von etwa 3:1 bis etwa 1:1 eingesetzt. Als Nachteil dieser Arbeitsweise wird die Bildung von Kupferfilmen beschrieben, die bei der destillativen Reinigung der Säure auftreten und mechanische Schäden in der Destillationsapparatur zur Folge haben. Um dieses Problem zu vermeiden wird empfohlen, die Destillation in Gegenwart von Sauerstoff durchzuführen.

Gegenstand der bekanntgemachten deutschen Patentanmeldung 26 04 545 ist die Herstellung von Alkylcarbonsäuren der allgemeinen Formel CₙH₂ₙ₊₁COOH, in der n einen Wert von 2 bis 18 bedeutet, durch Hydroformylierung, auch bekannt unter der Bezeichnung Oxosynthese, eines Olefins der allgemeinen Formel CₙH₂ₙ und durch unmittelbare Oxidation des bei der Hydroformylierung anfallenden Reaktionsgemisches. Unmittelbar heißt in diesem Zusammenhang, daß die vorherige Aufarbeitung des Hydroformylierungsgemischs unterbleibt und die nachfolgende Oxidationsreaktion in Gegenwart von Rhodium erfolgt. Das bekannte Oxidationsverfahren dient insbesondere zur Herstellung von Gemischen isomerer C₉-C₁₆-Fettsäuren. Als Ausgangsolefine für die Oxosynthese kommen bevorzugt die Dimere und Trimere des Propens und der Butene in Betracht, darunter vor allem das dimere Isobuten (2,4,4-Trimethylpenten-1). Beide Einzelumsetzungen des zweistufigen Verfahrens, d.h. sowohl die Hydroformylierung als auch die Oxidation, werden durch Rhodium in Form seiner Verbindungen katalysiert. Maßgebend für die Rhodium-Konzentration in dem der Oxidation unterworfenen Reaktionsgemisch ist daher der relativ hohe Rhodiumanteil im Hydroformylierungsprodukt. Um die Wirtschaftlichkeit des Gesamtprozesses sicherzustellen ist es erforderlich, das Edelmetall durch geeignete Maßnahmen möglichst vollständig aus dem Endprodukt des Prozesses, der Carbonsäure, wiederzugewinnen. Überdies ist nicht auszuschließen, dass durch Rhodium in der vorliegenden Konzentration während des Oxidationsvorganges unerwünschte Nebenreaktionen begünstigt werden, denn die Carbonsäure-Ausbeute ist, wie die Beispiele zeigen, für eine technische Nutzung des Verfahrens unzureichend.

LARKIN berichtet in J.Org.Chem. 1990, 55, S. 1563 ff., dass die Gegenwart von Katalysatoren bei der kommerziell ausgeführten Oxidation von Aldehyden zu Carbonsäuren deshalb für notwendig erachtet wird, weil im Reaktionsgemisch Spuren von Metallsalzen enthalten sind, die Nebenreaktionen katalysieren können. Die Bildung der Metallsalze geht auf die Korrosion metallischer Anlagenteile zurück. Aufgabe der Katalysatoren ist es, die Wirkung der Korrosionsprodukte zu überkompensieren.

Auch in Ullmanns Encyklopädie der technischen Chemie, 4. Auflage 1975, Band 9, wird mehrfach auf den negativen Einfluss metallischer Verunreinigungen in den zur Oxidation eingesetzten Ausgangsaldehyden hingewiesen. So führen z.B. im Butyraldehyd gelöste Eisen- und Kobaltsalze bei dessen Oxidation zu Buttersäure zu einem erhöhten Anfall von Nebenprodukten (1.c., Seite 142, linke Spalte) und bei der Oxidation von 2-Ethylhexanal zu 2-Ethylhexansäure beschleunigen Schwermetallionen die Decarbonylierung des Ausgangsaldehyds zu Heptan (1.c., Seite 144, linke Spalte).

Es gibt Hinweise im Stand der Technik, nach denen die Wirkung von Katalysatorzusätzen von der Struktur des für die Oxidation eingesetzten Aldehyds abhängt. So ist beispielsweise aus DE 950 007 bekannt, dass die Oxidation von in α-Stellung verzweigten Aldehyden den Zusatz geringer Mengen von carbonsauren Alkalisalzen erfordert, um die gewünschten Carbonsäuren in hoher Ausbeute bei gleichzeitig hoher Reinheit zu erhalten.

Nach der Lehre der offengelegten japanischen Patentanmeldung 53-105413 oxidiert man α-verzweigte aliphatische Aldehyde mit Sauerstoff in Gegenwart von Lithium- oder Erdalkalimetallverbindungen, die in Mengen von 0,01 bis 10 Gew.-% (bezogen auf das gesamte Reaktionssystem) eingesetzt werden, um α-verzweigte aliphatische Carbonsäuren herzustellen.

Kennzeichnend für die in der französischen Patentanmeldung 2 769 624 beschriebene Arbeitsweise ist die Einhaltung niedriger Reaktionstemperaturen, nämlich Temperaturen zwischen 0 und 25°C. Das Verfahren erfordert ebenfalls die Gegenwart von Alkalimetall- bzw. Erdalkalimetallverbindungen als Hilfsstoffe. Es bleibt offen, welche speziellen Wirkungen diese Verbindungen entfalten, d.h. ob sie, wie bekannt, lediglich die Selektivität der Umsetzung verbessern oder aber bei den gewählten niedrigen Temperaturen, möglicherweise auch die Reaktionsgeschwindigkeit erhöhen.

Bei der Oxidation von α-verzweigten Aldehyden, bei denen das dem Carbonylkohlenstoffatom benachbarte Kohlenstoffatom die Verzweigung trägt, empfiehlt somit der Stand der Technik den Zusatz geringer Mengen an Alkalimetallcarboxylaten zur Selektivitätsverbesserung. Ein derartiger Zusatz ist jedoch aufgrund ihrer inhibierenden Wirkung mit einer Verlängerung der Reaktionszeit verbunden. Unter den α-verzweigten Aldehyden besitzt besonders 2-Ethylhexanal, das in großen Mengen zur 2-Ethylhexansäure weiterverarbeitet wird, eine wirtschaftliche Bedeutung.

Die Oxidation von Aldehyden, die in β-Position, das heißt an dem, bezogen auf das Carbonylkohlenstoffatom, übernächsten Kohlenstoffatom die Verzweigung tragen, kann ebenfalls unter Katalysatorzusatz erfolgen. Einen wirtschaftlich bedeutenden Aldehyd mit einem hohen Anteil an β -alkylverzweigten Verbindungen erhält man durch Hydroformylierung von technisch verfügbarem Diisobuten (2,4,4-Trimethylpenten-1). Die Oxidation in Gegenwart von Rhodium nach DE-A1-26 04 545 liefert ein Gemisch isomerer C₉-Fettsäuren mit einem hohen Anteil an 3,5,5-Trimethylhexansäure, häufig auch als Isononansäure bezeichnet. Die Oxidation von β-alkylverzweigten Aldehyden, beispielsweise von lsovaleraldehyd, in Gegenwart von Alkali- oder Erdalkalicarboxylaten ist aus DE-A1-732 720 bekannt.

Lineare Aldehyde lassen sich nach der Lehre gemäß DE-C1-100 10 771 in Gegenwart von Übergangsmetallen oder deren Verbindungen in die entsprechenden Carbonsäuren überführen.

Die Patentschrift DE-C1-100 10 771 offenbart ebenfalls den Einsatz eines Gemisches aus Alkalisalzen und Übergangsmetallen bei der Oxidation von 2-Methylbutanal als α-verzweigten Aldehyd.

Aus GB 856,962 ist ein Oxidationsverfahren zur Herstellung von gesättigten aliphatischen Monocarbonsäuren bekannt, bei dem in Gegenwart eines Gemisches aus Kobalt- und/oder Mangansalzen mit Alkali- oder Erdalkalisalzen gearbeitet wird, wobei der Alkali- oder Erdalkalizusatz dem oxidativen Abbau entgegenwirkt. Beispielhaft wird die Oxidation von Propionaldehyd beschrieben, wobei Kobalt oder Mangan oder ein Gemisch davon in einer Menge von 0,00001 bis 1,0 mol je mol Propionaldehyd zugegen sind.

DE 10 2004 055 252 A1 behandelt die katalytische Oxidation von aliphatischen geradkettigen und von β-alkylverzweigten Aldehyden mit 5 bis 13 Kohlenstoffatomen zu den entsprechenden Carbonsäuren. Kennzeichnend für dieses katalytische Oxidationsverfahren ist der Einsatz eines Katalysatorsystems aus einem Gemisch aus Alkali- oder Erdalkalimetallcarboxylaten in einer Menge von 1-10 mmol, berechnet als Alkali- oder Erdalkalimetall, je Mol eingesetztem Aldehyd, und von 0,1 bis 5,0 ppm eines Metalls der Gruppen 5 bis 11 des Periodensystems der Elemente oder seiner Verbindungen oder eines Gemisches solcher Metalle sowie seiner Verbindungen. Die ppm-Angaben, berechnet als Übergangsmetall, beziehen sich auf eingesetzten Aldehyd.

Nach der Lehre der EP 1 854 778 A1 lässt sich das Oxidationsverfahren gemäß der DE 10 2004 055 252 A1 bezüglich Aldehydumsatz bei hoher Selektivität zu den entsprechenden Carbonsäuren nochmals verbessern, wenn das Katalysatorsystem aus dem Gemisch aus Alkali- oder Erdalkalimetallcarboxylaten und Übergangsmetallen der Gruppen 5 bis 11 des Periodensystems der Elemente nicht als Frischkatalysator, sondern als Umsetzungsprodukt aus einer Aldehydoxidationsreaktion eingesetzt wird. Die Herstellung dieses Umsetzungsprodukts in einer Aldehydoxidationsreaktion kann man auch als Präformierung oder Aktivierung des Katalysatorsystems ansehen. Nach Beendigung der Aldehydoxidationsreaktion werden die gebildete Carbonsäure, unumgesetzter Ausgangsaldehyd sowie weitere flüchtige Bestandteile destillativ abgetrennt und der das Umsetzungsprodukt enthaltende Destillationsrückstand für die eigentliche Aldehydoxidationsreaktion eingesetzt.

Nach EP 1 854 778 A1 ist der vorteilhafte Effekt der Präformierung des Katalysatorsystems auf das Oxidationsverhalten von Aldehyden nicht nur auf ein Katalysatorsystem, enthaltend Alkali- oder Erdalkalimetallcarboxylate und Übergangsmetalle der Gruppen 5 bis 11 des Periodensystems beschränkt, sondern wird auch bei Katalysatorsystemen beobachtet, die Metalle oder Metallverbindungen der Gruppen 4 und 12 des Periodensystems der Elemente sowie Cer oder Lanthan enthalten.

Es wurde nun gefunden, dass man bei der Herstellung des Umsetzungsprodukts aus der Aldehydoxidationsreaktion auch mit einer Menge an Alkali- oder Erdalkalimetallen und an katalytisch wirksamen Metallen ausgewählt aus der Gruppe von Titan, Vanadium, Chrom, Molybdän, Eisen, Kobalt, Nickel, Ruthenium, Rhodium, Palladium, Kupfer und Zink arbeiten kann, die bis zu der fünffachen, vorzugsweise bis zu der dreifachen Metallmenge beträgt, die bei dem unmittelbaren Einsatz des Umsetzungsprodukts aus der Aldehydoxidationsreaktion für die nachfolgende eigentliche Aldehydoxidationsreaktion erforderlich ist. Bei der Ausführung des erfindungsgemäßen Verfahrens wird nach destillativer Abtrennung der gebildeten Carbonsäure, des unumgesetzten Restaldehyds sowie weiterer flüchtiger Bestandteile, der angefallene Destillationsrückstand nicht insgesamt, sondern in einer solchen Menge für die nachfolgende, eigentliche Aldehydoxidationsreaktion eingesetzt, bei der der Metallgehalt in dem zu oxidierenden Aldehyd in dem Bereich liegt, den man einstellt, wenn man das Umsetzungsprodukt aus der Aldehydoxidationsreaktion unmittelbar für die nachfolgende, eigentliche Aldehydoxidationsreaktion einsetzt.

Die vorliegende Erfindung betrifft daher ein katalytisches Verfahren zur Herstellung von aliphatischen geradkettigen und von β-alkylverzweigten Carbonsäuren mit 5 bis 13 Kohlenstoffatomen durch Oxidation der entsprechenden Aldehyde mit Sauerstoff oder Sauerstoff enthaltenden Gasgemischen bei 20 bis 100°C in der Flüssigphase, **dadurch gekennzeichnet, dass** die Oxidation der Aldehyde in Gegenwart eines Katalysatorsystems erfolgt, das das Umsetzungsprodukt aus einer Aldehydoxidationsreaktion ist, in der die Aldehydoxidationsreaktion in Gegenwart von Alkali- oder Erdalkalimetallcarboxylaten oder eines Gemisches davon in einer Menge, berechnet als Alkali- oder Erdalkalimetall, von jeweils 2,5 mmol bis jeweils 25 mmol je mol eingesetztem Aldehyd, und von 0,25 bis 25 ppm eines Metalls ausgewählt aus der Gruppe von Titan, Vanadium, Chrom, Molybdän, Eisen, Kobalt, Nickel, Ruthenium, Rhodium, Palladium, Kupfer und Zink oder der entsprechenden Menge einer Verbindung eines solchen Metalls oder eines Gemisches solcher Metalle und/oder Metallverbindungen, bezogen auf eingesetzten Aldehyd, erfolgt, und das in einer solchen Menge für die Oxidation der Aldehyde eingesetzt wird, dass die Oxidation der Aldehyde in Gegenwart von Alkali- oder Erdalkalimetallcarboxylaten oder eines Gemisches davon in einer Menge, berechnet als Alkali- oder Erdalkalimetall, von jeweils 0,5 mmol bis jeweils 5 mmol je mol eingesetztem Aldehyd, und von 0,05 bis 5,0 ppm eines Metalls ausgewählt aus der Gruppe von Titan, Vanadium, Chrom, Molybdän, Eisen, Kobalt, Nickel, Ruthenium, Rhodium, Palladium, Kupfer und Zink oder der entsprechenden Menge einer Verbindung eines solchen Metalls oder eines Gemisches solcher Metalle und/oder Metallverbindungen, bezogen auf eingesetzten Aldehyd, erfolgt.

Vorzugsweise betragen die eingesetzten Mengen an Alkali- oder Erdalkalimetallcarboxylaten, berechnet als Alkali- oder Erdalkalimetall, jeweils 1,5 mmol bis jeweils 15 mmol je mol eingesetztem Aldehyd bei der Herstellung des Umsetzungsprodukts aus der Aldehydoxidationsreaktion. Die katalytisch aktiven Metalle ausgewählt aus der Gruppe von Titan, Vanadium, Chrom, Molybdän, Eisen, Kobalt, Nickel, Ruthenium, Rhodium, Palladium, Kupfer und Zink sind vorzugsweise in einer Menge von 0,15 bis 15 ppm, bezogen auf eingesetzten Aldehyd, zugegen.

Überraschenderweise gelingt es, aliphatische geradkettige oder β -alkylverzweigte Aldehyde mit reinem Sauerstoff oder Sauerstoff enthaltenden Gasgemischen bei hoher Selektivität und gleichzeitig erhöhtem Umsatz zu den entsprechenden Carbonsäuren umzusetzen, wenn man in Gegenwart eines Katalysatorsystems arbeitet, das geringe Mengen an Alkali- oder Erdalkalimetallcarboxylaten und geringe Mengen ausgewählter Metalle oder Verbindungen dieser Metalle enthält, das das Umsetzungsprodukt aus einer Aldehydoxidationsreaktion ist.

Wesentliches Merkmal des neuen Verfahrens ist der Einsatz eines Katalysatorsystems, enthaltend Alkali- oder Erdalkalimetallcarboxylate oder eines Gemisches davon und die katalytisch wirksamen Metalle in Form ihrer Umsetzungsprodukte aus einer Aldehydoxidationsreaktion. Es hat sich überraschenderweise gezeigt, dass ein Katalysatorsystem, das in Form seines Umsetzungsproduktes aus einer Aldehydoxidation eingesetzt wird, bei hoher Selektivität zu der gewünschten Carbonsäure eine deutliche Umsatzsteigerung bei der Aldehydoxidation bewirkt, so dass insgesamt höhere Ausbeuten an den gewünschten Carbonsäuren erzielt werden, gegenüber einem Verfahren, in dem mit einem Frischkatalysatorsystem, hergestellt aus Metallen, vorzugsweise in feinverteilter Form, oder aus handelsüblichen Metallsalzen, beispielsweise aus Acetaten, Acetylacetonaten, Metallcarbonylen, Carbonaten, Halogeniden, Sulfaten oder aus Metalloxiden, gearbeitet wird.

Die Herstellung des Katalysatorsystems in Form seines Umsetzungsprodukts erfolgt in einer separaten Aldehydoxidationsreaktion, wobei vorzugsweise der Aldehyd oxidiert wird, der auch in der eigentlichen Oxidationsstufe als Ausgangsprodukt dient. Die Herstellung dieses Umsetzungsprodukts und die eigentliche Oxidationsreaktion erfolgen ebenfalls vorzugsweise unter gleichen Reaktionsbedingungen, wobei der Einsatz unterschiedlicher Aldehyde und die Einstellung unterschiedlicher Reaktionsbedingungen bei der Herstellung des Umsetzungsprodukts und der davon separaten eigentlichen Oxidationsreaktion nicht ausgeschlossen sind.

Nach erfolgter Katalysatorpräformierung und destillativer Abtrennung von Carbonsäure, Restaldehyd und flüchtigen Komponenten, wird von dem angefallenen Destillationsrückstand eine Teilmenge für die eigentliche Aldehydoxidationsreaktion eingesetzt. Diese Teilmenge ist so bemessen, dass, die vorliegenden Mengen an Alkali- oder Erdalkalimetallcarboxylaten und an Übergangsmetallen ausgewählt aus der Gruppe von Titan, Vanadium, Chrom, Molybdän, Eisen, Kobalt, Nickel, Ruthenium, Rhodium, Palladium, Kupfer und Zink, bezogen auf zu oxidierenden Aldehyd, den Mengen und bevorzugten Mengen entsprechen, die man verwendet, wenn man das Umsetzungsprodukt aus der Aldehydoxidationsreaktion unmittelbar für die nachfolgende, eigentliche Aldehydoxidationsreaktion einsetzt.

Den bei der destillativen Aufarbeitung des rohen Carbonsäuregemischs aus der nachfolgenden, eigentlichen Aldehydoxidationsreaktion anfallenden Katalysator haltigen Rückstand kann man anschließend als Katalysator für die nächste Aldehydoxidationsreaktion wieder einsetzen. Eine besondere Reinigung des Katalysatorsystems für den Wiedereinsatz ist nicht erforderlich.

Es ist nicht erforderlich, die Alkali- oder Erdalkalimetallcarboxylate als einheitliche Verbindung einzusetzen. Es ist ebenfalls möglich, Gemische dieser Verbindungen sowie Gemische von Alkali- und Erdalkalimetallcarboxylaten, zu verwenden, wobei man jedoch zweckmäßigerweise die Carboxylate der bei der Oxidation entstehenden Carbonsäuren verwendet. Vorzugsweise setzt man jedoch einheitliche Verbindungen ein, beispielsweise Lithium-, Kalium-, Natrium-, Magnesium-, Calcium- oder Bariumcarboxylate, wie zum Beispiel Kaliumisononanoat, Natriumisononanoat, Calciumisononanoat, Bariumisononanoat, Kaliumpentanoat, Natriumpentanoat, Caiciumpentanoat oder Bariumpentanoat.

Im Allgemeinen stellt man eine Alkali- oder Erdalkalimetallcarboxylat enthaltende Lösung durch Neutralisation einer wässrigen die Alkali- oder Erdalkalimetallverbindung enthaltenden Lösung mit einem Überschuß an der jeweils gewünschten Carbonsäure her und setzt diese Lösung bei der Herstellung des Umsetzungsprodukts aus der Aldehydoxidationsreaktion dem zu oxidierenden Aldehyd zu. Als Alkali- oder Erdalkalimetallverbindungen eignen sich besonders die Hydroxide, Carbonate oder Hydrogencarbonate.

Es ist aber auch möglich, die Alkali- oder Erdalkalimetallcarboxylate im Reaktionsgemisch zu erzeugen, indem man ihm Alkali- oder Erdalkalimetallverbindungen zusetzt, die unter den Reaktionsbedingungen in die Carboxylate überführt werden. Beispielweise lassen sich Alkali- oder Erdalkalimetallhydroxide, -carbonate, -hydrogencarbonate oder -oxide in dem erfindungsgemäßen Verfahren einsetzen. Ihr Zusatz kann entweder in fester Form oder als wässrige Lösung erfolgen.

Bei der Herstellung des Umsetzungsprodukts aus der Aldehydoxidationsreaktion wird dem Oxidationsgemisch neben dem Alkali- oder Erdalkalimetallcarboxylat erfindungsgemäß mindestens ein Metall ausgewählt aus der Gruppe von Titan, Vanadium, Chrom, Molybdän, Eisen, Kobalt, Nickel, Ruthenium, Rhodium, Palladium, Kupfer und Zink oder mindestens eine Verbindung eines solchen Metalls, zugesetzt. Verwendet man Metalle in elementarer Form als Katalysatoren, so empfiehlt es sich, sie in feiner Verteilung dem Reaktionsgemisch hinzuzufügen. Statt der Metalle in elementarer Form können auch Verbindungen der Metalle als Katalysatoren Anwendung finden. Die Art der Verbindungen unterliegt dabei keiner Beschränkung. Sofern nicht besondere Gründe vorliegen, wird man jedoch solche Verbindungen bevorzugen, die im Reaktionsmedium von Anfang an löslich sind, um eine Verzögerung des Reaktionseintritts durch vorherige Bildung einer löslichen und damit besonders aktiven Metallverbindung zu vermeiden.

Zu den katalytisch, bereits in sehr geringer Menge wirksamen Metallen zählen Titan, Vanadium, Chrom, Molybdän, Eisen, Kobalt, Nickel, Ruthenium, Rhodium, Palladium, Kupfer, Zink, vorzugsweise Chrom, Eisen, Kupfer, Zink und insbesondere Titan, Eisen und Chrom. Als im Reaktionsgemisch lösliche Verbindungen verwendet man Salze, insbesondere Salze organischer Säuren, wobei Carboxylate der Säuren bevorzugt werden, die das Ergebnis der separaten, eigentlichen Aldehydoxidationsreaktion sind. Andere geeignete Verbindungen der erfindungsgemäß eingesetzten Metalle sind Komplexverbindungen, z.B. Acetylacetonate, Metallcarbonyle oder handelsübliche Metallsalze wie Acetate, Carbonate, Halogenide, Sulfate oder Metalloxide oder Metallalkoholate.

Für die Herstellung des Umsetzungsprodukts aus der Aldehydoxidationsreaktion ist es nicht erforderlich, die katalytisch wirksamen Metalle oder die katalytisch wirksamen Metalle enthaltenden Verbindungen als reine Substanzen einzusetzen. Vielmehr können auch Gemische der genannten Metalle bzw. der Metallverbindungen und ebenso auch Gemische aus Metallen und Metallverbindungen für die Herstellung des präformierten Katalysatorsystems eingesetzt werden.

Bei der Herstellung des Umsetzungsprodukts aus der Aldehydoxidationsreaktion ist ein maximales Gewichtsverhältnis zwischen Metall und zu oxidierendem Aldehyd einzuhalten. Die beschriebenen Verhältnisse zwischen Katalysatormetall und Aldehyd gelten auch bei Verwendung von Metallverbindungen, d.h. die Menge der einzusetzenden Verbindung bemisst sich nach ihrem Metallgehalt. Entsprechendes gilt bei Einsatz von Mischungen verschiedener katalytisch wirksamer Metalle bzw. Metallverbindungen und von Mischungen aus Metallen und Metallverbindungen.

Die Herstellung des Umsetzungsprodukts aus der Aldehydoxidationsreaktion erfolgt in einem Temperaturbereich von 20 bis 100°C. Vorzugsweise arbeitet man zwischen 20 und 80°C, insbesondere zwischen 40 und 80°C. Die Temperaturführung, konstante oder variable Temperatur, kann den individuellen Erfordernissen des Ausgangsmaterials und den Reaktionsgegebenheiten angepaßt werden.

Die Umsetzung der Reaktionspartner erfolgt bevorzugt bei Atmosphärendruck. Die Anwendung erhöhten Drucks ist jedoch nicht ausgeschlossen. Üblicherweise arbeitet man in einem Bereich von Atmosphärendruck bis 1,0 MPa, vorzugsweise bei Atmosphärendruck bis 0,8 MPa.

Die zur Herstellung des Umsetzungsprodukts aus der Aldehydoxidationsreaktion erforderliche Reaktionszeit hängt unter anderem ab von deren Reaktionstemperatur, der Art der Einsatzstoffe und dem Mengenverhältnis der Reaktanten zueinander. Normalerweise beträgt sie 30 Minuten bis 20 Stunden, insbesondere 2 bis 8 Stunden.

Anschließend wird das Reaktionsgemisch destilliert und die bereits erhaltene, gewünschte Carbonsäure abgetrennt. Ebenfalls destillativ abgetrennte Restmengen von Aldehyd lassen sich in der eigentlichen, separaten Aldehydoxidationsreaktion wieder einsetzen. Bei der Destillation wird im Allgemeinen soweit eingeengt, dass der anfallende Destillationsrückstand, der das Umsetzungsprodukt aus der Aldehydoxidationsreaktion, also das aktivierte Katalysatorsystem, gelöst enthält, noch technisch handhabbar ist, beispielsweise noch ausreichend pumpfähig ist.

Das im Destillationsrückstand enthaltene Umsetzungsprodukt aus der Aldehydoxidationsreaktion wird anschließend in der eigentlichen Oxidationsreaktion dem zu oxidierenden Aldehyd, der vorzugsweise der gleiche Aldehyd ist, der bei der Herstellung des Umsetzungsprodukts aus der Aldehydoxidationsreaktion verwendet wird, zugesetzt.

Die angewandten Metallmengen sowie die Gewichtsverhältnisse zwischen Metall und zu oxidierendem Aldehyd entsprechen dabei den zuvor beschriebenen Mengen und Verhältnissen.

Die angewandten Metallmengen stellen eine, auch für technische Bedürfnisse, ausreichende Reaktionsgeschwindigkeit sicher. Sie geben jedoch nicht zu unerwünschten Nebenreaktionen Anlass, so dass die Aldehyde nahezu ausschließlich in die ihnen entsprechenden Carbonsäuren umgewandelt werden. Überdies sind die eingesetzten Metallmengen so gering, dass sie weder unter dem Aspekt der Wirtschaftlichkeit des Verfahrens, z.B. bei Verwendung teurer Edelmetalle, noch im Hinblick auf die für die verschiedenen Anwendungsgebiete geforderte Reinheit der Carbonsäuren, aus dem Reaktionsprodukt wiedergewonnen bzw. entfernt werden müssen.

Auch entsprechen die gewählten Temperatur- und Druckbereiche in der eigentlichen, nachfolgenden Aldehydoxidationsreaktion die den in der Herstellung der Umsetzungsprodukte aus der Aldehydoxidationsreaktion angewandten Bedingungen.

Man führt somit die Oxidation von aliphatischen geradkettigen und von β -alkylverzweigten Aldehyden mit 5 bis 13 Kohlenstoffatomen vorzugsweise bei Temperaturen im Bereich von 20 bis 80°C, insbesondere 40 bis 80°C, und bei Drücken im Bereich von Atmosphärendruck bis 1,0 MPa, vorzugsweise bei Atmosphärendruck bis 0,8 MPa durch.

Die erforderliche Reaktionszeit in der Oxidationsreaktion hängt unter anderem ab von der Reaktionstemperatur, der Art der Einsatzstoffe und dem Mengenverhältnis der Reaktanten zueinander. Normalerweise beträgt sie 30 Minuten bis 20 Stunden, insbesondere 2 bis 8 Stunden.

Als Oxidationsmittel verwendet man bei der Herstellung des Umsetzungsprodukts aus der Aldehydoxidationsreaktion und in der eigentlichen, separaten Aldehydoxidationsreaktion molekularen Sauerstoff oder Gasgemische, die molekularen Sauerstoff enthalten. Weitere Bestandteile derartiger Gasgemische sind inerte Gase, z.B. Stickstoff, Edelgase und Kohlendioxid. Der Anteil der inerten Bestandteile des Sauerstoff enthaltenden Gasgemisches beträgt bis zu 90 Vol-%, insbesondere 30 bis 80 Vol-%. Die bevorzugten Oxidationsmittel sind Sauerstoff oder Luft.

Die Aldehyde können als solche oder gelöst in einem, unter den Reaktionsbedingungen inerten Lösungsmittel eingesetzt werden. Beispiele für geeignete Lösungsmittel sind Ketone wie Aceton, Ester, z.B. Ethylacetat, Kohlenwasserstoffe, z.B. Toluol und Nitrokohlenwasserstoffe wie Nitrobenzol. Die Konzentration des Aldehyds wird durch seine Löslichkeit in dem Lösungsmittel begrenzt.

Sowohl der Präformierungsschritt des Katalysatorsystems als auch die eigentliche Aldehydoxidationsreaktion kann absatzweise oder kontinuierlich durchgeführt werden. Eine Rückführung nicht umgesetzter Reaktionsteilnehmer ist in beiden Fällen möglich.

Aus dem nach der Oxidation anfallenden Rohsäuregemisch wird mittels Destillation unter üblichen Bedingungen die reine Carbonsäure gewonnen. Der die Alkali- oder Erdalkalimetallcarboxylate und katalytische Metalle enthaltende Destillationsrückstand wird abgetrennt und kann dem Einsatzaldehyd, gegebenenfalls nach Zugabe von frischen Alkali- oder Erdalkalimetallcarboxylaten oder Alkali- oder Erdalkalimetallverbindungen, die unter den Reaktionsbedingungen in die Carboxylate übergehen, sowie von katalytischem Metall wieder zugeführt werden.

Nach einer bewährten Ausführungsform des erfindungsgemäßen Verfahrens legt man den Aldehyd in Gegenwart der Alkali- oder Erdalkalimetallcarboxylate zusammen mit dem katalytischen Metall in einem geeigneten Reaktor, z.B. in einem mit einem Anströmboden versehenen Rohrreaktor, der gegebenenfalls noch Füllkörper enthält, vor und leitet den Sauerstoff oder das Sauerstoff enthaltende Gasgemisch von unten durch den Aldehyd. In dem Präformierungsschritt des Katalysatorsystems wird analog gearbeitet.

Entsprechend einer weiteren Ausführungsform verwendet man als Reaktor einen Rieselturm, der Füllkörper enthält. Über die Füllung läßt man den Alkali- oder Erdalkalimetallcarboxylate sowie katalytisches Metall enthaltenden Aldehyd herabrieseln und leitet in den Turm gleichzeitig im Gleich- oder Gegenstrom, Sauerstoff oder ein Sauerstoff enthaltendes Gasgemisch ein.

Im Mittelpunkt des neuen Prozesses steht die Oxidation von aliphatischen geradkettigen oder β-alkylverzweigten Aldehyden mit 5 bis 13 Kohlenstoffatomen. Unter β-alkylverzweigten Aldehyden sind auch solche Aldehyde zu verstehen, die neben der β-Alkylverzweigung weitere Seitengruppen an dem Kohlenstoffatomgerüst tragen. Die Herkunft der Aldehyde ist nicht auf bestimmte Herstellungsverfahren beschränkt. Aufgrund ihrer leichten Zugänglichkeit werden durch Oxosynthese, d.h. durch Reaktion von Olefinen mit 4 bis 12 Kohlenstoffatomen mit Kohlenmonoxid und Wasserstoff gewonnene Aldehyde bevorzugt. In diesem Zusammenhang ist es nicht entscheidend, welche spezielle Ausführungsform der Oxosynthese zur Gewinnung der Aldehyde diente, d.h. ob die Reaktion z.B. durch Kobalt oder durch Rhodium katalysiert wurde, ob man die Metalle allein oder zusammen mit Komplexbildnern einsetzte und der Katalysator im Reaktionsgemisch homogen gelöst war oder eine eigene, heterogene Phase bildete.

Besonders eignet sich das erfindungsgemäße Verfahren zur Herstellung von Isononansäure aus dem Reaktionsprodukt der mit Diisobutylen durchgeführten Oxosynthese. Das technisch verfügbare Reaktionsprodukt der Oxosynthese von Diisobutylen enthält als Hauptbestandteil 3,5,5- Trimethylhexanal sowie geringe Mengen an 3,4,4-, und 3,4,5-Trimethylhexanal. Darüber hinaus sind geringe Mengen an nicht in β-Position verzweigten Aldehyden, wie 2,5,5-Trimethylhexanal, 4,5,5-Trimethylhexanal und 6,6-Dimethylheptanal zugegen. Die Oxidation dieses technisch verfügbaren Gemisches isomerer Nonanale nach dem erfindungsgemäßen Verfahren führt bei einer ausgezeichneten selektiven Bildung von Isononansäure zu einer deutlichen Umsatzsteigerung.

Ebenso ist das erfindungsgemäße Verfahren für die Oxidation von n-Pentanal, n-Heptanal, n-Nonanal sowie Isovaleraldehyd, zu den entsprechenden Carbonsäuren in hervorragender Weise geeignet.

In den folgenden Beispielen wird die Herstellung von n-Pentansäure nach dem beanspruchten Verfahren beschrieben.

Die Herstellung des Umsetzungsprodukts aus der Aldehydoxidationsreaktion erfolgt entsprechend der Erfindung in Gegenwart von Alkali- oder Erdalkalimetallcarboxylaten und von den katalytischen Metallen durch Sauerstoffoxidation des zu oxidierenden Aldehyds. Nach destillativer Abtrennung der flüchtigen Anteile wird der angefallene Destillationsrückstand als Katalysatorsystem für die nachfolgende Aldehydoxidationsreaktion eingesetzt.

Der nach der eigentlichen Aldehydoxidationsreaktion anfallende Katalysatorrückstand wird für nachfolgende Aldehydoxidationsreaktionen wieder verwendet.

Die jeweiligen Versuchsergebnisse werden durch die Angaben folgender Kenngrößen wiedergegeben:
- Aldehyd-Umsatz;
- Selektivität, sie ergibt sich aus dem Carbonsäure-Anteil im Reaktionsprodukt, bezogen auf umgesetzten Aldehyd;
- Ausbeute an Carbonsäure

### Beispiel

### Teilweiser Einsatz des Umsetzungsprodukts aus der Aldehydoxidationsreaktion für die Aldehydoxidationsreaktion.

### Durchführung:

Die Flüssigphasenoxidation von Aldehyd zu Carbonsäure erfolgte in einem Blasensäulenreaktor aus Glas mit einem Innendurchmesser von 38 mm und 150 cm Länge. Abhängig vom Reaktionsverhalten wurde der Reaktor mantelseitig durch einen mit einem Wärmetauscher verbundenen Wasserkreislauf gekühlt oder beheizt und die Innentemperatur auf diese Weise konstant gehalten. Die Sauerstoffzufuhr erfolgte von unten durch eine mit der Blasensäule verbundene Glasfilterplatte mit einer maximalen Porenweite von 16-40 µm.

Nach Abschluss der Reaktion wurde die Rohsäure komplett an einer einfachen Vakuumdestillationsapparatur bei einem Druck von 100 hPa destilliert, der verbleibende Rückstand mit frischem Aldehyd gemischt und die so anfallende Lösung nachfolgend oxidiert.

### Versuchsauswertung:

Die in den GC-Analysen aufgeführten Werte sind Flächenprozente und beinhalten unter dem Punkt Carbonsäure auch den jeweils bereits im Einsatz befindlichen Anteil. Da die Einsatzmengen an Aldehyd und Carbonsäure mit 760,0 g bzw. 40,0 g stets konstant gehalten wurden, ist ein Vergleich der GC-Analysen im Verlauf einer Versuchsreihe aber zulässig.

Bei den Angaben zum Umsatz, der Selektivität sowie der Ausbeute ist die eingesetzte Menge an Carbonsäure herausgerechnet, somit beziehen sich diese Angaben nur auf das Resultat der Oxidation, nämlich dem jeweiligen Aldehydumsatz sowie der dabei gebildeten Menge an Carbonsäure.

### Beispiel : Herstellung von n-Pentansäure

- Ausgangsaldehvd GC-Analyse:: 0,06 % Vorlaufkomponenten
0,64 % Isopentanal
99,15 % n-Pentanal
0,10 % n-Pentansäure
0,05 % Sonstige

### a) Herstellung des Umsetzungsprodukts aus der Aldehydoxidationsreaktion; Einsatz von Frischkatalysator (Kombination Eisen/Kalium)

Der Oxidationseinsatz bestand aus einem homogenen Gemisch von 760,0 g n-Pentanal, 26,5 g n-Pentansäure mit einem Gehalt von 3,8 mg Eisen (eingetragen als Eisen(II)-acetat) sowie 22,95 g einer Lösung bestehend aus 6,20 g Kalium-n-pentanoat, 13,50 g n-Pentansäure und 3,25 g Wasser.

Nach 2,5 Stunden Oxidation bei konstant 50°C und einem Gesamteinsatz von 106 Liter Sauerstoff (gemessen bei 20°C) wurde eine Rohsäure der folgenden Zusammensetzung erhalten:
0,50 % Vorlaufkomponenten
0,04 % Isopentanal
8,66 % n-Pentanal
1,20 % Komponenten
0,56 % Isopentansäure
88,08 % n-Pentansäure
0,96 % Nachlaufkomponenten

Der Umsatz (bezogen auf n-Pentanal) betrug 89,4 % der Theorie, die zugehörige Selektivität zur Bildung von n-Pentansäure 96,1 % der Theorie. Daraus lässt sich eine Ausbeute von 85,9 % berechnen.

### b) Eigentliche Aldehydoxidationsreaktion: Katalysatorwiedereinsatz

Die im zuvor beschriebenen Versuch hergestellte Rohsäure wurde destilliert, wobei der den Katalysator enthaltende Rückstand in einer Menge von 41,0 g anfiel.

Vom Destillationsrückstand wurden 8,2 g (darin enthalten 1,2 g Kalium-n-pentanoat) zusammen mit 760,0 g n-Pentanal und 33,0 g n-Pentansäure in die Oxidation zurückgeführt.

Die Oxidation erfolgte unter den bereits genannten Bedingungen (2,5 Stunden Reaktionszeit, 50°C Reaktionstemperatur, 106 Liter Sauerstoff (gemessen bei 20°C). Es wurde eine Rohsäure der folgenden Zusammensetzung erhalten:
0,16 % Vorlaufkomponenten
0,03 % Isopentanal
5,26 % n-Pentanal
0,35 % Komponenten
0,57 % Isopentansäure
93,02 % n-Pentansäure
0,61 % Nachlaufkomponenten

Der Aldehydumsatz betrug 93,5 %, die Ausbeute 92,4 %.

Auch die Arbeitsweise, bei der man zunächst die Katalysatorpräformierung in Gegenwart höherer Metallmengen vornimmt und anschließend einen Teil des Katalysatorsystems für die eigentliche, nachfolgende Aldehydoxidationsreaktion einsetzt, führt zu einer deutlichen Steigerung in der Carbonsäureausbeute.

## Patentansprüche

1. Katalytisches Verfahren zur Herstellung von aliphatischen geradkettigen und von β-alkylverzweigten Carbonsäuren mit 5 bis 13 Kohlenstoffatomen durch Oxidation der entsprechenden Aldehyde mit Sauerstoff oder Sauerstoff enthaltenden Gasgemischen bei 20 bis 100°C in der Flüssigphase, **dadurch gekennzeichnet, dass** die Oxidation der Aldehyde in Gegenwart eines Katalysatorsystems erfolgt, das das Umsetzungsprodukt aus einer Aldehydoxidationsreaktion ist, in der die Aldehydoxidationsreaktion in Gegenwart von Alkali- oder Erdalkalimetallcarboxylaten oder eines Gemisches davon in einer Menge, berechnet als Alkali- oder Erdalkalimetall, von jeweils 2,5 mmol bis jeweils 25 mmol je mol eingesetztem Aldehyd, und von 0,25 bis 25 ppm eines Metalls ausgewählt aus der Gruppe von Titan, Vanadium, Chrom, Molybdän, Eisen, Kobalt, Nickel, Ruthenium, Rhodium, Palladium, Kupfer und Zink oder der entsprechenden Menge einer Verbindung eines solchen Metalls oder eines Gemisches solcher Metalle und/oder Metallverbindung, bezogen auf eingesetzten Aldehyd, erfolgt, und dass nach destillativer Abtrennung der gebildeten Carbonsäure, des unumgesetzten Restaldehyds sowie weiterer flüchtiger Bestandteile der angefallenen Destillationsrückstand in einer solchen Menge für die Oxidation der Aldehyde eingesetzt wird, dass die Oxidation der Aldehyde in Gegenwart von Alkali- oder Erdalkalimetallcarboxylaten oder eines Gemisches davon in einer Menge, berechnet als Alkali- oder Erdalkalimetall, von jeweils 0,5 mmol bis jeweils 5 mmol je mol eingesetztem Aldehyd, und von 0,05 bis 5,0 ppm eines Metalls ausgewählt aus der Gruppe von Titan, Vanadium, Chrom, Molybdän, Eisen, Kobalt, Nickel, Ruthenium, Rhodium, Palladium, Kupfer und Zink oder der entsprechenden Menge einer Verbindung eines solchen Metalls oder eines Gemisches solcher Metalle und/oder Metallverbindungen, bezogen auf eingesetzten Aldehyd, erfolgt.

2. Verfahren nach Anspruch 1, **dadurch gekennzeichnet, dass** die Herstellung des Umsetzungsprodukts aus der Aldehydoxidationsreaktion in Gegenwart von Alkali- oder Erdalkalimetallcarboxylaten oder eines Gemisches davon in einer Menge, berechnet als Alkali- oder Erdalkalimetall, von jeweils 1,5 bis jeweils 15 mmol je mol eingesetztem Aldehyd und in Gegenwart von 0,15 bis 15 ppm eines Metalls ausgewählt aus der Gruppe von Titan, Vanadium, Chrom, Molybdän, Eisen, Kobalt, Nickel, Ruthenium, Rhodium, Palladium, Kupfer und Zink oder der entsprechenden Menge einer Verbindung eines solchen Metalls oder eines Gemisches solcher Metalle und/oder Metallverbindungen, bezogen auf eingesetzten Aldehyd, erfolgt.

3. Verfahren nach Anspruch 1 oder 2, **dadurch gekennzeichnet, dass** man als Alkalimetallcarboxylate Lithium, Natrium oder Kaliumcarboxylate verwendet und dass man als Erdalkalimetallcarboxylate Magnesium, Calcium und Bariumcarboxylate verwendet.

4. Verfahren nach Anspruch 3, **dadurch gekennzeichnet, dass** die Alkalimetallcarboxylate oder Erdalkalimetallcarboxylate Salze der Carbonsäuren sind, die als Ergebnis in der Aldehydoxidationsreaktion entstehen.

5. Verfahren nach einem oder mehreren der Ansprüche 1 bis 4, **dadurch gekennzeichnet, dass** die Metallverbindungen Carboxylate, Acetylacetonate, Metallcarbonyle, Acetate, Carbonate, Halogenide, Sulfate, Metalloxide oder Metallalkoholate sind.

6. Verfahren nach Anspruch 5, **dadurch gekennzeichnet, dass** die Metallcarboxylate Salze der Carbonsäuren sind, die als Ergebnis in der Aldehydoxidationsreaktion entstehen.

7. Verfahren nach einem oder mehreren der Ansprüche 1 bis 6, **dadurch gekennzeichnet, dass** die Herstellung des Umsetzungsprodukts aus der Aldehydoxidationsreaktion bei Temperaturen im Bereich von 20 bis 100°C, vorzugsweise 20 bis 80°C, und insbesondere zwischen 40 und 80°C, durchgeführt wird.

8. Verfahren nach einem oder mehreren der Ansprüche 1 bis 7, **dadurch gekennzeichnet, dass** die Herstellung des Umsetzungsprodukts aus der Aldehydoxidationsreaktion bei Drücken in einem Bereich von Atmosphärendruck bis 1,0 MPa, vorzugsweise Atmosphärendruck bis 0,8 MPa durchgeführt wird.

9. Verfahren nach einem oder mehreren der Ansprüche 1 bis 8, **dadurch gekennzeichnet, dass** bei der Herstellung des Umsetzungsprodukts aus der Aldehydoxidationsreaktion die Sauerstoff enthaltenden Gasgemische einen Anteil von bis zu 90 Vol.-%, insbesondere 30 bis 80 Vol.-%, inerter Bestandteile aufweisen.

10. Verfahren nach einem oder mehreren der Ansprüche 1 bis 9, **dadurch gekennzeichnet, dass** die Oxidation von aliphatischen geradkettigen und von β-alkylverzweigten Aldehyden mit 5 bis 13 Kohlenstoffatomen bei Temperaturen im Bereich von 20 bis 80°C, vorzugsweise 40 bis 80°C, und bei Drücken im Bereich von Atmosphärendruck bis 1,0 MPa, vorzugsweise bei Atmosphärendruck bis 0,8 MPa durchgeführt wird.

11. Verfahren nach einem oder mehreren der Ansprüche 1 bis 10, **dadurch gekennzeichnet, dass** die bei der Oxidation von aliphatischen geradkettigen und von β-alkylverzweigten Aldehyden mit 5 bis 13 Kohlenstoffatomen eingesetzten Sauerstoff enthaltenden Gasgemische einen Anteil von bis zu 90 Vol.-%, insbesondere 30 bis 80 Vol.-%, inerter Bestandteile aufweisen.

12. Verfahren nach einem oder mehreren der Ansprüche 1 bis 11, **dadurch gekennzeichnet, dass** bei der Herstellung des Umsetzungsprodukts aus der Aldehydoxidationsreaktion der zu oxidierende aliphatische geradkettige oder β-alkylverzweigte Aldehyd mit 5 bis 13 Kohlenstoffatomen eingesetzt wird.

## Claims

1. A catalytic process for preparing aliphatic straight-chain and β-alkyl-branched carboxylic acids having from 5 to 13 carbon atoms by oxidation of the corresponding aldehydes by means of oxygen or oxygen-containing gas mixtures at from 20 to 100°C in the liquid phase, wherein the oxidation of the aldehydes is carried out in the presence of a catalyst system which is the reaction product from an aldehyde oxidation reaction in which the aldehyde oxidation reaction is carried out in the presence of alkali metal carboxylates or alkaline earth metal carboxylates or a mixture thereof in an amount, calculated as alkali or alkaline earth metal, of from 2.5 mmol to 25 mmol per mol of aldehyde used and of from 0.25 to 25 ppm of a metal selected from the group consisting of titanium, vanadium, chromium, molybdenum, iron, cobalt, nickel, ruthenium, rhodium, palladium, copper and zinc or the corresponding amount of a compound of such a metal or a mixture of such metals and/or metal compounds, based on the aldehyde used and, after removal by distillation of the carboxylic acid formed, of the unreacted residual aldehyde and further volatile constituents of the distillation residue obtained, is used in the oxidation of the aldehydes in such an amount that the oxidation of the aldehydes is carried out in the presence of alkali metal carboxylates or alkaline earth metal carboxylates or a mixture thereof in an amount, calculated as alkali or alkaline earth metal, of from 0.5 mmol to 5 mmol per mol of aldehyde used and of from 0.05 to 5.0 ppm of a metal selected from the group consisting of titanium, vanadium, chromium, molybdenum, iron, cobalt, nickel, ruthenium, rhodium, palladium, copper and zinc or the corresponding amount of a compound of such a metal or a mixture of such metals and/or metal compounds, based on the aldehyde used.

2. The process as claimed in claim 1, wherein the preparation of the reaction product from the aldehyde oxidation reaction is carried out in the presence of alkali metal carboxylates or alkaline earth metal carboxylates or a mixture thereof in an amount, calculated as alkali or alkaline earth metal, of from 1.5 mmol to 15 mmol per mol of aldehyde used and in the presence of from 0.15 to 15 ppm of a metal selected from the group consisting of titanium, vanadium, chromium, molybdenum, iron, cobalt, nickel, ruthenium, rhodium, palladium, copper and zinc or the corresponding amount of a compound of such a metal or a mixture of such metals and/or metal compounds, based on the aldehyde used.

3. The process as claimed in claim 1 or 2, wherein lithium, sodium or potassium carboxylates are used as alkali metal carboxylates and magnesium, calcium and barium carboxylates are used as alkaline earth metal carboxylates.

4. The process as claimed in claim 3, wherein the alkali metal carboxylates or alkaline earth metal carboxylates are salts of the carboxylic acids which are formed as a result in the aldehyde oxidation reaction.

5. The process as claimed in one or more of claims 1 to 4, wherein the metal compounds are carboxylates, acetylacetonates, metal carbonyls, acetates, carbonates, halides, sulfates, metal oxides or metal alkoxides.

6. The process as claimed in claim 5, wherein the metal carboxylates are salts of the carboxylic acids which are formed as a result in the aldehyde oxidation reaction.

7. The process as claimed in one or more of claims 1 to 6, wherein the preparation of the reaction product from the aldehyde oxidation reaction is carried out at temperatures in the range from 20 to 100°C, preferably from 20 to 80°C and in particular from 40 to 80°C.

8. The process as claimed in one or more of claims 1 to 7, wherein the preparation of the reaction product from the aldehyde oxidation reaction is carried out at pressures in a range from atmospheric pressure to 1.0 MPa, preferably from atmospheric pressure to 0.8 MPa.

9. The process as claimed in one or more of claims 1 to 8, wherein, in the preparation of the reaction product from the aldehyde oxidation reaction, the oxygen-containing gas mixtures have a proportion of up to 90% by volume, in particular from 30 to 80% by volume, of inert constituents.

10. The process as claimed in one or more of claims 1 to 9, wherein the oxidation of aliphatic straight-chain and β-alkyl-branched aldehydes having from 5 to 13 carbon atoms is carried out at temperatures in the range from 20 to 80°C, preferably from 40 to 80°C, and at pressures in the range from atmospheric pressure to 1.0 MPa, preferably at from atmospheric pressure to 0.8 MPa.

11. The process as claimed in one or more of claims 1 to 10, wherein the oxygen-containing gas mixtures used in the oxidation of aliphatic straight-chain and β-alkyl-branched aldehydes having from 5 to 13 carbon atoms have a proportion of up to 90% by volume, in particular from 30 to 80% by volume, of inert constituents.

12. The process as claimed in one or more of claims 1 to 11, wherein the aliphatic straight-chain or β-alkyl-branched aldehyde having from 5 to 13 carbon atoms which is to be oxidized is used in the preparation of the reaction product from the aldehyde oxidation reaction.

## Revendications

1. Procédé catalytique de fabrication d'acides carboxyliques aliphatiques linéaires et β-alkylramifiés contenant 5 à 13 atomes de carbone par oxydation des aldéhydes correspondants avec de l'oxygène ou des mélanges gazeux contenant de l'oxygène à une température de 20 à 100 °C en phase liquide, **caractérisé en ce que** l'oxydation des aldéhydes a lieu en présence d'un système catalytique qui est le produit de réaction d'une réaction d'oxydation d'aldéhydes, la réaction d'oxydation d'aldéhydes ayant lieu en présence de carboxylates de métaux alcalins ou alcalino-terreux ou d'un mélange d'entre eux en une quantité, calculée en tant que métal alcalin ou alcalino-terreux, de respectivement 2,5 mmol à respectivement 25 mmol par mol d'aldéhyde utilisé, et de 0,25 à 25 ppm d'un métal choisi dans le groupe constitué par le titane, le vanadium, le chrome, le molybdène, le fer, le cobalt, le nickel, le ruthénium, le rhodium, le palladium, le cuivre et le zinc ou la quantité correspondante d'un composé d'un tel métal ou d'un mélange de tels métaux et/ou composés métalliques, par rapport à l'aldéhyde utilisé, et **en ce qu'**après une séparation par distillation de l'acide carboxylique formé, de l'aldéhyde résiduel non réagi et d'autres constituants volatils du résidu de distillation formé sont utilisés pour l'oxydation des aldéhydes en une quantité telle que l'oxydation des aldéhydes ait lieu en présence de carboxylates de métaux alcalins ou alcalino-terreux ou d'un mélange d'entre eux en une quantité, calculée en tant que métal alcalin ou alcalino-terreux, de respectivement 0,5 mmol à respectivement 5 mmol par mol d'aldéhyde utilisé, et de 0,05 à 5,0 ppm d'un métal choisi dans le groupe constitué par le titane, le vanadium, le chrome, le molybdène, le fer, le cobalt, le nickel, le ruthénium, le rhodium, le palladium, le cuivre et le zinc ou la quantité correspondante d'un composé d'un tel métal ou d'un mélange de tels métaux et/ou composés métalliques, par rapport à l'aldéhyde utilisé.

2. Procédé selon la revendication 1, **caractérisé en ce que** la fabrication du produit de réaction de la réaction d'oxydation d'aldéhydes a lieu en présence de carboxylates de métaux alcalins ou alcalino-terreux ou d'un mélange d'entre eux en une quantité, calculée en tant que métal alcalin ou alcalino-terreux, de respectivement 1,5 à respectivement 15 mmol par mol d'aldéhyde utilisé, et en présence de 0,15 à 15 ppm d'un métal choisi dans le groupe constitué par le titane, le vanadium, le chrome, le molybdène, le fer, le cobalt, le nickel, le ruthénium, le rhodium, le palladium, le cuivre et le zinc ou la quantité correspondante d'un composé d'un tel métal ou d'un mélange de tels métaux et/ou composés métalliques, par rapport à l'aldéhyde utilisé.

3. Procédé selon la revendication 1 ou 2, **caractérisé en ce que** des carboxylates de lithium, de sodium ou de potassium sont utilisés en tant que carboxylates de métaux alcalins et **en ce que** des carboxylates de magnésium, de calcium et de baryum sont utilisés en tant que carboxylates de métaux alcalino-terreux.

4. Procédé selon la revendication 3, **caractérisé en ce que** les carboxylates de métaux alcalins ou les carboxylates de métaux alcalino-terreux sont des sels des acides carboxyliques formés en tant que produits lors de la réaction d'oxydation d'aldéhydes.

5. Procédé selon une ou plusieurs des revendications 1 à 4, **caractérisé en ce que** les composés métalliques sont des carboxylates, des acétylacétonates, des carbonyles de métaux, des acétates, des carbonates, des halogénures, des sulfates, des oxydes de métaux ou des alcoolates de métaux.

6. Procédé selon la revendication 5, **caractérisé en ce que** les carboxylates de métaux sont des sels des acides carboxyliques formés en tant que produits lors de la réaction d'oxydation d'aldéhydes.

7. Procédé selon une ou plusieurs des revendications 1 à 6, **caractérisé en ce que** la fabrication du produit de réaction de la réaction d'oxydation d'aldéhydes est réalisée à des températures dans la plage allant de 20 à 100 °C, de préférence de 20 à 80 °C, et notamment comprises entre 40 et 80 °C.

8. Procédé selon une ou plusieurs des revendications 1 à 7, **caractérisé en ce que** la fabrication du produit de réaction de la réaction d'oxydation d'aldéhydes est réalisée à des pressions dans une plage allant de la pression atmosphérique à 1,0 MPa, de préférence de la pression atmosphérique à 0,8 MPa.

9. Procédé selon une ou plusieurs des revendications 1 à 8, **caractérisé en ce que** lors de la fabrication du produit de réaction de la réaction d'oxydation d'aldéhydes, les mélanges gazeux contenant de l'oxygène présentent une proportion de constituants inertes de jusqu'à 90 % en volume, notamment de 30 à 80 % en volume.

10. Procédé selon une ou plusieurs des revendications 1 à 9, **caractérisé en ce que** l'oxydation d'aldéhydes aliphatiques linéaires et β-alkylramifiés contenant 5 à 13 atomes de carbone est réalisée à des températures dans la plage allant de 20 à 80 °C, de préférence de 40 à 80 °C, et à des pressions dans la plage allant de la pression atmosphérique à 1,0 MPa, de préférence de la pression atmosphérique à 0,8 MPa.

11. Procédé selon une ou plusieurs des revendications 1 à 10, **caractérisé en ce que** les mélanges gazeux contenant de l'oxygène utilisés lors de l'oxydation d'aldéhydes aliphatiques linéaires et β-alkylramifiés contenant 5 à 13 atomes de carbone présentent une proportion de constituants inertes de jusqu'à 90 % en volume, notamment de 30 à 80 % en volume.

12. Procédé selon une ou plusieurs des revendications 1 à 11, **caractérisé en ce que** l'aldéhyde aliphatique linéaire ou β-alkylramifié contenant 5 à 13 atomes de carbone à oxyder est utilisé lors de la fabrication du produit de réaction de la réaction d'oxydation d'aldéhydes.
